(19) Europäisches Patentamt
European Patent Office
Office européen des brevets

(11) EP 2 725 359 B1

(12) **EUROPEAN PATENT SPECIFICATION**

(45) Date of publication and mention of the grant of the patent:
**27.05.2015 Bulletin 2015/22**

(51) Int Cl.:
***G01N 33/532*** *(2006.01)* ***G01N 33/543*** *(2006.01)*
***A61K 47/48*** *(2006.01)*

(21) Application number: **13188639.2**

(22) Date of filing: **15.10.2013**

(54) **Cell separation method using a release system for cell-antibody-substrate conjugates containing a polyethylene glycol spacer unit**

Zelltrennungsverfahren mit einem Freisetzungssystem für Zell-Antikörper-Substrat-Konjugate mit einer Polyethylenglycol-Distanzeinheit

Procédé de séparation de cellule utilisant un système de libération pour conjugués cellule-anticorps-substrat contenant une unité d'espacement de polyéthylène glycol

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.10.2012 EP 12189516**

(43) Date of publication of application:
**30.04.2014 Bulletin 2014/18**

(73) Proprietor: **Miltenyi Biotec GmbH**
**51429 Bergisch Gladbach (DE)**

(72) Inventors:
- **Dose, Christian, Dr.**
  **D-51429 Bergisch Gladbach (DE)**
- **Brieden, Jennifer**
  **D-51429 Bergisch Gladbach (DE)**

(74) Representative: **Kisters, Michael Marcus**
**Patentanwalt**
**Miltenyi Biotec GmbH**
**Corporate Legal Department**
**Friedrich-Ebert Strasse 68**
**51429 Bergisch Gladbach (DE)**

(56) References cited:
**US-A- 5 332 679** **US-A- 5 518 882**

- **SHAN KE ET AL: "Avidin-Biotin-PEG-CPA Complexes as Potential EPR-Directed Therapeutic Protein Carriers: Preparation and Characterization", BIOCONJUGATE CHEMISTRY, vol. 18, no. 5, 1 September 2007 (2007-09-01), pages 1644-1650, XP055050030, ISSN: 1043-1802, DOI: 10.1021/bc700182t**
- **KE S ET AL: "Intermolecular interaction of avidin and PEGylated biotin", BIOCONJUGATE CHEMISTRY, ACS, WASHINGTON, DC, US, vol. 18, no. 6, 21 November 2007 (2007-11-21), pages 2109-2114, XP002573017, ISSN: 1043-1802, DOI: 10.1021/BC700204K [retrieved on 2007-10-19]**
- **RAMPERSAUD A A ET AL: "Novel discrete PEG-based crosslinking reagents for conjugation of antibodies and proteins to biotin, fluorochromes, enzymes and gold that eliminate aggregation, improves solubility, reduces non-specific binding and enhances low level detection limits", MOLECULAR BIOLOGY OF THE CELL, vol. 22, 2011, page 1931, XP055050022, & ANNUAL MEETING OF THE AMERICAN-SOCIETY-FOR-CELL-BIOLOGY (ASCB); DENVER, CO, USA; DECEMBER 03 -07, 2011 ISSN: 1059-1524**

EP 2 725 359 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Field of invention

The present invention relates to a cell separation method using a release system for cell-antibody-substrate conjugates including a method to selectively dissociate the conjugates.

### Background of the invention

The ability of biotin to bind streptavidin, avidin, and other biotin-binding molecules has been exploited for several decades, because of the high affinity, specificity, and broad applicability of this system.

To improve the release properties of biotin/streptavidin affinity systems, chemically modified biotin and streptavidin derivatives have been introduced, wherein only one or even both binding partners were modified. Such modifications lower the stability of the biotin/streptavidin complex by several orders of magnitude and thereby facilitate the dissociation of the two binding partners (see for example US 20080255004). Furthermore, mutated streptavidin proteins have been developed with reduced affinity for biotin or its analogues (M. Qureshi et al, J. Biol. Chem. 276 (2001) 46422-46428; T. Sano et al, Proc.Natl. Acad. Sci. USA 92 (1995) 3180-3184). US 5506121 discloses so called "strept-tags" i.e. peptides with reduced binding affinity to streptavidin.

The modification of biotin or streptavidin is laborious. Since many reagents for the coupling of biotin with substrates, like antibodies, are commercially available, it is widely spread to use unmodified biotin as labeling agent.

In this respect, US 5215927 describes the isolation of target cells by contacting the desired cell population with a monoclonal antibody and subsequent incubation with a biotinylated anti-species immunoglobulin directed to the specific monoclonal antibody. The mixture is separated over a solid phase comprising immobilized avidin as biotin-binding molecule, which facilitates the immobilization of the target cells and separation of unlabeled targets. The desired cells are subsequently released by mechanical agitation to disrupt the immobilized complex.

The use of release mechanisms mediated by unselective enzyme degradation, chemical reactions, intense mechanical forces, high temperature, or strong saline conditions are undesirable for the separation of living cells, because it is important to preserve the cells integrity and viability. Accordingly, mechanisms are desired that allow a rapid and selective release of the target cells at physiological conditions.

In alternative to enzymatic or chemical cleavage or the use of modified biotin/streptavidin molecules, biotin/streptavidin systems can also be cleaved by a ligand competition mechanism. For example, a biotinylated molecule can be released from a streptavidin-support by adding an excess of free biotin, thereby replacing the biotinylated molecule.

Methods based on the competition of free biotin or streptavidin against the respective counterpart (streptavidin or biotin) are known in numerous variants. James Hirsch et al. give an overview of these techniques in Analytical Biochemistry 308 (2002) 343-357.

Furthermore, Ke Shan et al. disclose in "Avidin-Biotin-PEG-CPA Complexes as Potential EPR-Directed Therapeutic Protein Carriers: Preparation and Characterization", Bioconjugate Chemistry, vol. 18, no. 5, 1 September 2007 (2007-09-01), pages 1644-1650 and "Intermolecular interaction of avidin and PEGylated biotin", Bioconjugate Chemistry, vol. 18, no. 6, 21 November 2007 (2007-11-21), pages 2109-2114, the use of biotin for dissociation of the Avidin-Biotin-PEG-enzyme-complex. A similar method is described by Rampersaudet al: "Novel discrete PEG-based crosslinking reagents for conjugation of antibodies and proteins to biotin, fluorochromes, enzymes and gold that eliminate aggregation, improves solubility, reduces non-specific binding and enhances low level detection limits" in MOLECULAR BIOLOGY OF THE CELL, vol. 22, 2011, page 1931.

The competition reaction of free biotin/streptavidin against the respective bound counterpart is disclosed in WO 92/16841 for analytical means. WO 92/16841 describes inter alia a method for detecting a reporter molecule, which is specifically bound to an analyte and an insoluble phase via a streptavidin/biotin-binding system. After the work-up procedure, the streptavidin/biotin binding system is cleaved by a displacement ligand and the released analyte is analytically detectable via the reporter molecule.

US 2008/0255004, US 6869606, and US 4656252 disclose biotinylated antibodies comprising modified biotin, wherein the biotin moiety and the antibody moiety of the biotinylated antibodies are separated by a spacer group consisting of an aryl, alkyl, or aminocaproic acid group. The use of hydrophobic aryl or alkyl groups is expected to cause agglomeration in aqueous solutions. Since physiological conditions of biological systems usually require aqueous solutions, agglomeration is an eminent problem for techniques utilizing rather hydrophobic substances. Spacer molecules derived from amino caproic acid (so called "LC linker") possess a linear alkyl chain with residues that support homo- or heterofunctional bioconjugation chemistries.

US 5518882 discloses a similar method, wherein target cells are bound to a solid support, for example magnetic particles. This allows a further enrichment by applying a magnetic field, which immobilizes the target cells coupled to the magnetic beads. The target cells can be released from the particles by cleaving the biotin-binding system with a

displacement ligand. Preferable, the conjugate "(magnetic bead)-antibiotin-biotin-antibody-cell" is cleaved by adding free streptavidin in access, resulting in a "(magnetic bead)-antibiotin" and a "streptavidin-biotin-antibody-cell" conjugate.

In general, a competition reaction, i.e. the displacement of a first ligand with a second ligand will only proceed until the equilibrium between the kinetics of the binding reaction of the first and second ligand is reached. The equilibrium depends on the respective binding forces and concentrations of the ligand and the thermodynamic conditions of the reaction. The known competition reactions to displace biotin by streptavidin therefore lead either to an uncompleted release or are difficult to control since the underlying kinetics are usually not known.

The present release systems are not fast or reliable enough for a process involving labeling of living cells, cell separation or detection and unlabeling of the target cells.

**Summary of the invention**

It was therefore an object of the present invention to provide a reliable and highly efficient release system comprising a biotinylated antibody for detection of living cells for use in a cell separation method.

Surprisingly, it was found that the binding affinity of a labeled binding partner targeted to the biotin moiety of a biotinylated antibody can be adjusted by placing a biocompatible spacer molecule between the biotin moiety and the antibody, wherein the labeled binding partner is provided with a detection means. With such a conjugate, cells can be first labeled for identification or isolation and after identification or isolation, the label can be removed to unlabel the desired target cells.

Object of the invention is therefore a method for the separation of target cells from a cell sample comprising the steps:

a) incubation of the cell sample with a releasable conjugate comprising a biotinylated ligand having a biotin moiety, a ligand moiety ($Ligand_1$) and a biotin-binding molecule (bbm) bound to the biotin moiety of the biotinylated ligand, wherein the biotin moiety and the ligand moiety of the biotinylated ligand are separated by a spacer group consisting of polyethylene glycol according to the general formula I

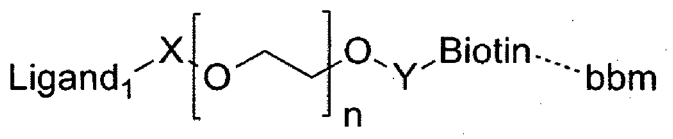

I

with n = 1 - 500, and X, Y = same or different, substituted alkyl groups having 1 to 20 carbon atoms with amine, amide, and/or thioether residues,

wherein the ligand is a first antibody for which the target cells express an antigen, and the biotin-binding molecule (bbm) is a second antibody which comprises a magnetic particle as solid support,

b) separation of the cells labeled with the a solid support obtained in step a) from the cell sample by applying an magnetic field,

c) adding a release agent in a sufficient concentration to displace the biotin-binding molecule (bbm) from the biotin moiety of the biotinylated ligand.

The conjugate of formula I is dissociated by treatment with a release agent, which disrupts the interaction between the biotin and the biotin-binding molecule.

The release system used in the invention is prepared by coupling a biotinylated ligand comprising a spacer group consisting of polyethylene glycol between the biotin and the ligand moiety with a biotin-binding molecule. The biotinylated ligand comprising the spacer group consisting of polyethylene glycol units might be coupled with one or several biotin-binding molecules and *vise versa* resulting in a releasable conjugate having one or more releasable cleavage sites. Accordingly, releasable conjugates used in the invention may comprise biotinylated ligand, especially biotinylated antibodies carrying 1 to 20 biotin-binding molecules (bbm).

Preferable, the number n of glycol units in the polyethylene spacer is between 1 and 100, more preferred between 1 and 25, and most preferred between 1 and 10.

In a first, second, and third embodiment of the invention, the releasable conjugate has the structure according to general formula IIa, IIb, or IIc. In these embodiments, n stands for the number of glycol units as already disclosed.

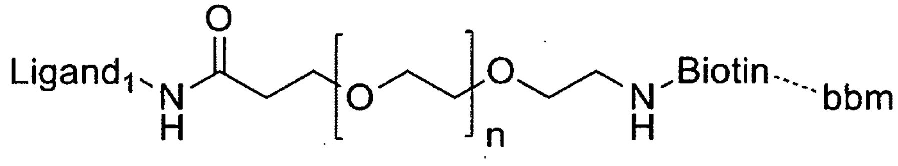

IIa

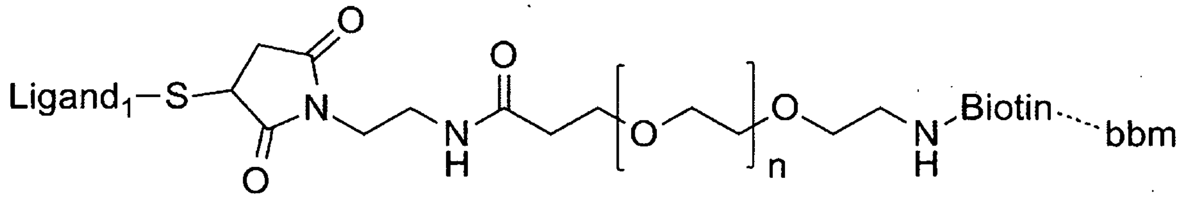

IIb

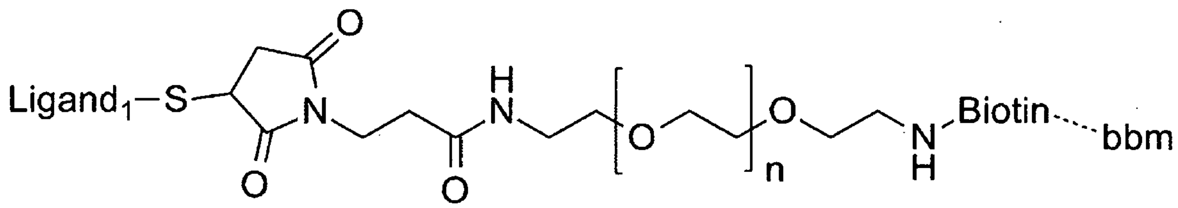

IIc

Fig.1 shows conjugates according to the prior art containing Biotin (1), LC-Biotin (2), LC-LC-Biotin (3) (not according to the invention), and PEO-Biotin (4) (according to the method of the invention). LC stands for "Long chain", a residue based on 6-aminocaproic acid; LC-LC stands for a double 6-aminocaproic acid spacer, while PEO stands for polyethylene oxide also known as polyethylene glycol.

The term "biotin" refers to 5-[(3a*S*,4*S*,6a*R*)-2-oxohexahydro-1*H*-thieno[3,4-*d*]imidazol-4-yl]pentanoic acid. The term "biotin" includes analogues or modified biotin compounds, which support similar host/guest interactions as unmodified biotin like, for example, iminobiotin, desthiobiotin, and DSB-X biotin. Preferable, unmodified biotin is used in the present invention.

The term "ligand" refers to any kind of antibody or fragmented antibody, directed against antigens expressed intracellular or extra cellular. The term relates to fully intact antibodies or fragmented antibody derivatives, e.g., Fab, Fab', F(ab')2, sdAb, scFv, di-scFv that have been synthesized by recombinant procedures including covalent and non-covalent conjugates containing these kind of molecules. Preferable, the term "ligand" refers to an antibody directed against antigen expressed by the target cells intracellular, like IL2, FoxP3, CD154, or extra cellular, like CD3, CD14, CD4, CD8, CD25, CD34, CD56, and CD133.

The term "biotin-binding molecule" (bbm) refers to an antibody, which is conjugated to a detection means and is displaceable from the biotin moiety of the biotinylated ligand by biotin or streptavidin. Preferable, the biotin-binding molecule (bbm) is an antibody selected from the class of immunoglobulin, e.g. IgG, IgA, IgM, IgD, IgE derived from animals, like e.g. mice, monkeys, goats, rabbits, sheep, or lamas. Furthermore, the term "biotin-binding molecule" (bbm) relates to fully intact or fragmented antibody derivatives, e.g., Fab, Fab', F(ab')2, sdAb, scFv, di-scFv that have been synthesized by recombinant procedures including covalent and non-covalent conjugates containing these kind of molecules.

The term "release agent" refers to any compound capable of binding to the biotin moiety of the biotinylated ligand or the biotin-binding molecule thereby disrupting the interaction between the biotin moiety and the biotin-binding molecule. Suitable release agents are streptavidin or biotin. The preferred release agent in this invention is biotin. Suitable-modified streptavidin molecules are disclosed by James Hirsch et al. in Analytical Biochemistry 308 (2002) 343-357. Accordingly, the term "streptavidin and derivates thereof" refers to any molecule derived from streptavidin providing a binding affinity to biotin that is comparable to the binding affinity of unmodified streptavidin to biotin as well as to derivatives and conjugates of streptavidin without having the purpose of especially lowering the binding affinity to

biotin.

The ligand, the biotin-binding molecule (bbm) and/or the release agent can be provided with a detection means, i.e. possess a label that can be used for detection. The detection means may for example emit a detection signal, like a chromophor unit, a fluorescence unit, or a radioactive unit. Suitable labeled release agents are, for example, commercial available under the trade names "anti-Biotin-PE" and "APC-Streptavidin" from Miltenyi Biotec GmbH, Germany and BioLegend Inc., respectively.

Furthermore, the detection means can be a solid-support, on which the biotin-binding molecule (bbm) and/or the ligand can be immobilized. The solid-support may be any of the known systems in biotechnology for immobilizing cells and can have the shape of particles, for example, sheets, plates, membranes, tubes, columns, wells, or micro arrays manufactured from various materials like polystyrene (PS), polymethylmethacrylate (PMMA), polyvinyl toluene (PVT), polyethylene (PE), or polypropylene (PP). Suitable materials are commercially available.

The solid support used in the method of the invention is a nano- to microscale magnetic particle, also known in the art as magnetic bead. The mean diameter of the beads can range from 10 nm to 10 $\mu$m. Biocompatible magnetic particles are commercially available and consist of, for example, forms of magnetically iron oxide coated by a shell of dextran molecules or silica. The solid support may also be polymers containing magnetic materials. Suitable particles are commercial available from Miltenyi Biotec GmbH, Germany under the trade name "MicroBeads" and "MACSiBeads" possessing a hydrodynamic diameter of 50-100 nm or 3-4 $\mu$m, respectively.

The use of the ligand, the biotin-binding molecule (bbm) and/or the release agent provided with a detection means allows a quantitative detection of the respective compound and/or of targeted cells and/or the separation or identification of the targeted cells.

According to this invention, magnetic particles are used as solid-support to facilitate magnetic separation processes. For example, the biotin-binding molecule (bbm) can be coupled to a magnetic particle via a covalent coupling procedure. In this respect, the magnetic particles would act as a solid-support in separation protocols. Suitable magnetic particles conjugated with, for example anti-biotin antibodies and apparatus for magnetic separations of living cells are available from Miltenyi Biotec GmbH, Germany.

In a variant of the invention, the detection means comprises an antibody, which is used to bind the biotin-binding molecule (bbm). Such antibody-coupled detection means, especially antibody-coupled magnetic beads are commercially available.

This variant of the invention is illustrated in general formula IV, with antibody$_1$ targeted to a biomolecule of interest, antibody$_2$ as biotin-binding molecule, and antibody$_3$, coupled to a detection means, like a solid support, binding to antibody$_2$.

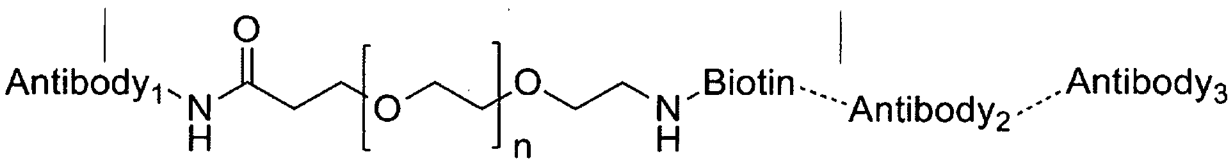

IV

Suitable magnetic beads coupled to anti-IgG antibodies are commercial available from Miltenyi Biotec GmbH as "Anti-IgG MicroBeads".

In the method of the invention, the releasable conjugate is cleaved by a release agent like biotin or streptavidin which is provided in a sufficient concentration to displace the biotin-binding molecule (bbm) from the biotin moiety of the biotinylated ligand.

All embodiments and variants of the already disclosed releasable conjugate can be employed in the method of the invention.

The method of the invention results in the dissociation of the releasable conjugate between the biotin moiety and the anti-biotin antibody as biotin-binding molecule (bbm) as shown in figures V and VI. The releasable conjugate is selectively cleaved between the biotin moiety and the anti-biotin antibody by adding an excess of, for example, free biotin as release agent. The release agent forms a non-covalent complex with the biotin-binding molecule (bbm) and facilitates the release of the biotinylated ligand (V).

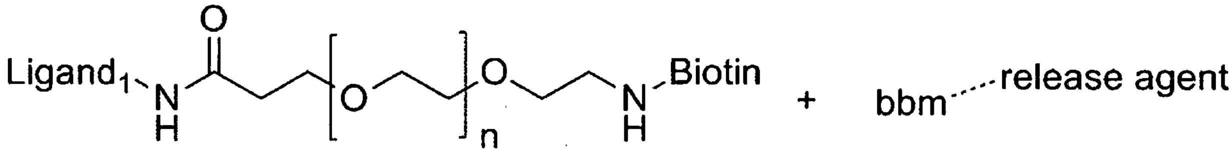

V

In alternative, the utilization of an excess of, e.g., streptavidin as release agent forms a non-covalent complex with the biotinylated ligand, which results in a displacement of the biotin-binding molecule (VI).

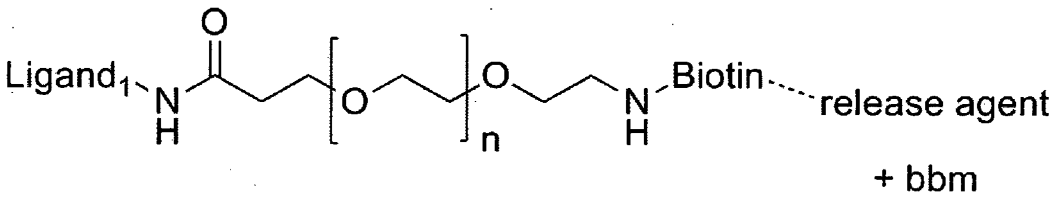

VI

The method of the invention is based on a competition reaction and will proceed until the binding equilibrium between the release agent, the biotin-binding molecule (bbm), and accordingly the biotin moiety is reached. The equilibrium depends on the respective binding forces, the concentration of the free release agent, and the thermodynamic conditions of the reaction. Accordingly, the term "sufficient amount to displace the second ligand by streptavidin or derivates thereof" does not stand for a specific value but needs to be evaluated according to the desired release rate.

For example, by utilizing an anti-biotin antibody as biotin-binding molecule (bbm) the releasable conjugate of the invention can be selectively cleaved by utilizing free biotin or streptavidin as release agent. If free biotin is used as release agent, a molar ratio (biotinylated ligand:free biotin) of 1:1,000 to 1:1,000,000, preferable a molar ratio of 1:1,000 to 1:100,000 is sufficient to dissociate the releasable conjugate. In case of streptavidin as release agent, a molar ratio (biotinylated ligand:streptavidin) of 1:1 to 1:10,000 preferable 1:10 to 1:1,000 may be provided to dissociate the releasable conjugate.

In another embodiment of the method of the invention, it was found that the addition of an auxiliary release agent improves the release efficiency method of the invention. In this embodiment, the release agent and an auxiliary release agent are provided in a sufficient concentration to displace the biotin-binding molecule (bbm) from the biotin moiety of the biotinylated ligand. Suitable auxiliary release agents have the general formulas VII, VIII, or IX

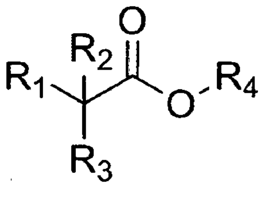

VII

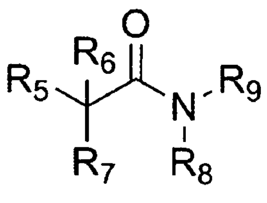

VIII

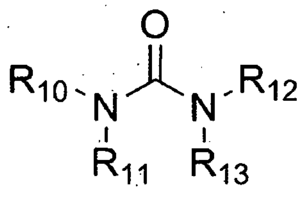

IX

with $R_{1\text{-}3,5\text{-}13}$ = same or different hydrogen, substituted, or unsubstituted alkyl residues having 1-20 carbon atoms and $R_4$ = substituted or unsubstituted alkyl residues having 1-20 carbon atoms.

The following compounds 7-15 illustrate specific examples of auxiliary release agents according to the invention. Not suitable as auxiliary release agent according to the invention is, for example, compound 15, despite its similar molecular structure in comparison to compounds 7 -14.

| No. | Chemical structure |
|---|---|
| 7 | 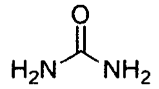 |
| 8 | 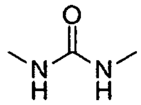 |
| 9 | 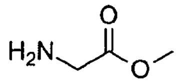 |
| 10 | 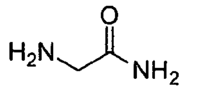 |
| 11 | 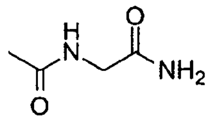 |
| 12 | 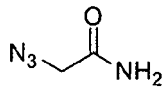 |
| 13 | 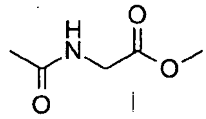 |
| 14 | 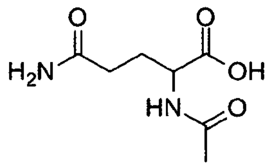 |
| 15 | 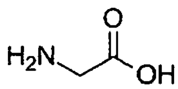 |

In this variant of the invention, the person skilled in the art can easily evaluate the suitable concentration of the auxiliary release agent with respect to the desired release rate. The examples provide some insights regarding suitable conditions and resulting release efficiencies. In praxis, the auxiliary release agent can be used in an high excess in comparison to the release agent, for example with a molar ratio of 1:1,000 to 1:1,000,000 (release agent:auxiliary release agent).

All variants of the already disclosed releasable conjugate can be employed in the method of the invention.

Optionally, subsequent to step c), in a further step d) the biotin-binding molecule (bbm) is separated from the target cells. The target cells may still be labeled with the conjugate according to the general formula I after separation.

The method according to the invention includes in step c) and optionally in step d) a separation of the labeled cells. To this end, the biotin-binding molecule of the releasable conjugate comprises a magnetic bead as solid support and the separation step is performed by applying a magnetic field. Magnetic cell sorting is known to the person skilled in the art and can be conducted in a permanent or an electromagnetic field with or without the use of a ferromagnetic column containing ferromagnetic material. Columns containing ferromagnetic material enhance the gradient of the magnetic field and are available from Miltenyi Biotec GmbH, Germany.

In the invention, the magnetically labeled cells are separated from the non-magnetic (i.e. non-labeled) cells by applying a magnetic field. After separation, the magnetic label can be removed by adding biotin or streptavidin with or without an auxiliary release agent in a sufficient concentration to displace the biotin-binding molecule (bbm) in step c).

This release step can be performed within or outside of the magnetic field. For example, the magnetic labeled

cells may be washed from the column and get the magnetic label removed outside of the magnetic field. In alternative, the magnetically labeled cells can be unlabeled by adding the release agent to the column located in the magnetic field. In this variant, the target cells (with the conjugate) are eluated from the column/the magnetic fields whereas the magnetic label remains on the column and in the magnetic field.

In the optional step d), i.e., the separation of the biotin-binding molecule (bbm) from target cells can be achieved by centrifugation or preferable by applying a magnetic field, i.e., by magnetic cell sorting technique as already described. Step c) and d) are preferable conducted in at least one (the same) or especially in two different columns containing ferromagnetic material.

Especially for the quantitative detection of the target cells within the method of the invention, the ligand and/or the biotin-binding molecule (bbm) may preferentially comprise a chromophor or a fluorescence unit as detection means. The detection means of the ligand and/or the biotin-binding molecule (bbm) is detected subsequent to step c) to detect the target cells. Methods for cell detection, for example, fluorescence activated cell sorting (FACS) are known to the person skilled in the art.

## Examples

The examples and comparative examples show that the method according to the invention allows a fast and reliable dissociation. Cells targeted by the releasable conjugates can be separated from cell suspensions and efficiently released, which enables a further processing of the isolated cells.

<u>Example 1 - Coupling of anti-CD8-Biotin conjugates and cell surface staining:</u>

Coupling protocol 1:

To prepare conjugates according to the first embodiment, IIa anti-CD8-antibody in PBS/EDTA-buffer was re-buffered to 100 mM $NaHCO_3$-buffer (pH 8.3). NHS-Biotin, NHS-LC-Biotin, NHS-LC-LC-Biotin, and NHS-PEO-Biotin (n=4, as shown in Fig.1, available from Thermo Scientific/Pierce) were dissolved in DMSO at concentration of 5 mg/mL and added in different molar ratios to the antibody solution at 2.5 mg/mL. After 1 h incubation time at room temperature, unreacted biotin was removed by gel filtration utilizing PBS/EDTA-buffer. Protein concentrations were determined by the absorbance at 280 nm. The approximate biotin to protein ratios (B/P) were determined by the standardized HABA-avidin-assay.

Coupling protocol 2:

To prepare bioconjugates according to the second embodiment IIb or IIc anti-CD8-antibody was reduced with 10 mM DTT in MES-buffer. After 1 h incubation time at room temperature, the antibody was purified by gel filtration utilizing PBS/EDTA-buffer. To afford the anti-CD8-$PEO_4$-Biotin (5) (according to IIb) and anti-CD8-$PEO_2$-Biotin (6) (according to IIc) conjugates maleimide-$PEO_4$-Biotin or maleimide-$PEO_2$-Biotin (available from Thermo Scientific/Pierce), respectively, were dissolved in PBS/EDTA-buffer at 5 mg/mL and added with a molar excess to the antibody solution at 2.5 mg/mL. After 15 h incubation at room temperature, the unreacted biotin was removed by gel filtration utilizing PBS/EDTA-buffer. Protein concentrations were determined by the absorbance at 280 nm. The approximate biotin to protein ratios (B/P) were determined by the standardized HABA-avidin-assay.

Cell surface staining with anti-CD8-Biotin conjugates: peripheral blood mononuclear cells (PBMCs) in PBS/EDTA/BSA-buffer were stained for 10 min at 4 °C with 0.5 μg/mL of anti-CD8-Biotin (1), anti-CD8-LC-Biotin (2), anti-CD8-LC-LC-Biotin (3), or anti-CD8-PEO-Biotin (4) as illustrated in Fig. 1. The cells were washed with cold PBS/EDTA/BSA-buffer and stained for 10 min at 4 °C with an excess of anti-Biotin-PE. The cells were washed with cold PBS/EDTA-BSA-buffer and analyzed by flow cytometry.

Fig. 2 shows the mean fluorescence intensities that have been achieved with different anti-CD8-Biotin conjugates as a function of the B/P ratio. Cell surface staining with anti-CD8-LC-Biotin (2), anti-CD8-LC-LC-Biotin (3), or anti-CD8-PEO-Biotin (4) furnished equally fluorescence signals. In contrast, cells stained with anti-CD8-Biotin (1) missing a spacer molecule between the biotin and the antibody moiety provided diminished mean fluorescence intensity although B/P ratio of the conjugates was comparable to the other biotinylated antibodies. These experiments illustrate the advantage of affinity systems containing spacer molecules as in anti-CD8-LC-Biotin (2), anti-CD8-LC-LC-Biotin (3), or anti-CD8-PEO-Biotin (4) in comparison to conjugates, like anti-CD8-Biotin (1) missing an additional linker between the biotin and antibody moiety.

Example 2 - Cell surface staining with anti-CD8-Biotin conjugates and kinetic analysis:

PBMCs in PBS/EDTA/BSA-buffer were stained for 10 min at 4 °C with 0.5 μg/mL of anti-CD8-Biotin (1), anti-CD8-LC-Biotin (2), anti-CD8-LC-LC-Biotin (3), anti-CD8-PEO-Biotin (4), anti-CD8-PEO-Biotin (5), or anti-CD8-$PEO_2$-Biotin (6). The cells were washed with cold PBS/EDTA/BSA-buffer and stained for 10 min at 4 °C with an excess of anti-Biotin-PE. The cells were washed with cold PBS/EDTA-BSA-buffer and incubated with 0.1 mM biotin at room temperature in the dark. The cells and the dissociation of anti-Biotin-PE was monitored by flow cytometry analysis after certain time points in relation to the starting sample missing release agent.

Fig. 3 illustrate a fast decrease of the fluorescence signal of CD8 positive cells within the first 10 min reaction time in the presence of anti-CD8-Biotin (1), anti-CD8-PEO-Biotin (4), anti-CD8-PEO-Biotin (5), or anti-CD8-$PEO_2$-Biotin (6), while the dissociation rate in the case of anti-CD8-LC-Biotin (2) and anti-CD8-LC-LC-Biotin (3) was significant slower. These examples show that the release of conjugates comprising either no spacer molecule or spacer units according to the invention is much faster than with conjugates comprising a spacer molecule not according to the invention, like LC or LC-LC. It is important to note, that spacer molecules seem to be necessary to achieve equally cell stainings as already disclosed in Fig. 2.

Example 3 - Cell surface staining with anti-CD8-PEO-Biotin conjugate and kinetic analysis:

CD8 positive cells in PBS/EDTA/BSA-buffer were stained for 10 min at 4 °C with 0.5 μg/mL anti-CD8-PEO-Biotin (4). The cells were washed with cold PBS/EDTA/BSA-buffer and stained 10 min at 4 °C with an excess of anti-Biotin-PE. The cells were washed with cold PBS/EDTA-BSA-buffer and incubated with different concentrations of the compounds 10, 12, and 15 at room temperature in the dark. The dissociation of the anti-CD8-PEO-Biotin/anti-Biotin-PE system was monitored by flow cytometry analysis after 10 min incubation time in relation to samples missing release agent.

Fig. 4 illustrates that the compounds 10 and 12 highly facilitate the dissociation of the anti-CD8-PEO-Biotin/anti-Biotin-PE system in comparison to molecule 15, furnishing lower release yields. These examples show that auxiliary release agents according to the invention can facilitate the dissociation of the invented release.

Example 4 - Cell surface staining with anti-CD8-Biotin conjugates and kinetic analysis:

CD8 positive cells in PBS/EDTA/BSA-buffer were stained for 10 min at 4 °C with 0.5 μg/mL anti-CD8-LC-LC-Biotin (3) and anti-CD8-PEO-Biotin (4). The cells were washed with cold PBS/EDTA/BSA-buffer and stained for 10 min at 4 °C with an excess of anti-Biotin-PE. The cells were washed with cold PBS/EDTA-BSA-buffer and incubated with different amounts of streptavidin in absence or in combination with compound 12 at 100 mM concentration. The dissociation of the anti-CD8-PEO-Biotin/anti-Biotin-PE and the anti-CD8-LC-LC-Biotin/anti-Biotin-PE systems were monitored by flow cytometry analysis after 10 min incubation times in relation to samples missing release agent.

Fig. 5 shows that the anti-CD8-PEO-Biotin/anti-Biotin-PE system dissociates significantly faster than the anti-CD8-LC-LC-Biotin/anti-Biotin-PE system in the presence of streptavidin. Moreover, the addition of compound 12 as auxiliary release agent accelerates the streptavidin-mediated dissociation process. This example shows that streptavidin on its own and in combination with compound 12 as auxiliary release agent can facilitate the dissociation of conjugates containing a spacer molecule according to the invention. In contrast, the dissociation of affinity systems possessing a spacer molecule not according to the invention, like LC-LC, is significantly less influenced by the utilization of the release agents.

Example 5 - Cell surface staining with anti-CD8-Biotin conjugates and kinetic analysis:

CD8 positive cells in PBS/EDTA/BSA-buffer were stained for 10 min at 4 °C with 0.5 μg/mL of anti-CD8-LC-LC-Biotin (3) and anti-CD8-PEO-Biotin (4). The cells were washed with cold PBS/EDTA/BSA-buffer and stained for 10 min at 4 °C with an excess of anti-Biotin-PE. The cells were washed with cold PBS/EDTA-BSA-buffer and incubated with different concentrations of allophycocyanin (APC)-labeled streptavidin (Biolegend, Inc.) in absence or in combination with compound 12. The dissociation of the anti-CD8-PEO-Biotin/anti-Biotin-PE and the anti-CD8-LC-LC-Biotin/anti-Biotin-PE systems were monitored by flow cytometry analysis after 10 min incubation times in relation to samples missing release agent.

Fig. 6 shows that the anti-CD8-PEO-Biotin/anti-Biotin-PE system dissociates significantly faster than the anti-CD8-LC-LC-Biotin/anti-Biotin-PE system in the presence of APC-streptavidin. Moreover, the addition of compound 12 as auxiliary release agent accelerates the APC-streptavidin-mediated dissociation process. This example shows that APC-streptavidin on its own and in combination with compound 12 as auxiliary release agent can facilitate the dissociation of releasable conjugates containing a spacer molecule according to the invention. In contrast, the release agents significantly less influence the dissociation of affinity systems possessing a spacer molecule not according to the invention,

like the anti-CD8-LC-LC-Biotin/anti-Biotin-PE system.

Example 6 - Magnetic cell separation with anti-CD8-PEO-Biotin conjugate:

PBMCs in PBS/EDTA/BSA-buffer were incubated at 4 °C for 10 min with anti-CD8-LC-LC-Biotin (3) and anti-CD8-PEO-Biotin (4). The cells were washed with cold PBS/EDTA/BSA-buffer and incubated for 15 min at 4 °C with anti-Biotin-MicroBeads (Miltenyi Biotec GmbH) and for 5 min at 4 °C with anti-CD8-PE. The cells were washed with cold PBS/EDTA-BSA-buffer and resuspended in 500 μL of cold buffer. The cell suspension was applied on a MS-column (Miltenyi Biotec GmbH) in a magnetic field for magnetic cell separation. The flow-through was collected as magnetically unlabeled cell fraction. The cells were washed within the magnetic field and the column was removed from the separator prior to the elution of the cells with 1 mL of cold PBS/EDTA/BSA-buffer. The isolated cell fraction was incubated for 10 min at different concentrations of biotin. The cell suspension was applied onto a second column and flow-through was collected as eluted cells. Cells retained on the column were eluted with 500 μL of cold PBS/EDTA/BSA-buffer in absence of the magnetic field. The amount of CD8 positive cells in the isolated fractions was determined by flow cytometry analysis. The percentage allocation of the eluted cells was calculated as follows:

$$\frac{\text{amount of cells in the eluted fraction}}{\text{amount of cells in the eluted fraction} + \text{amount of cells in the fraction retained by magnetic field}}$$

Fig. 7 illustrates that the incubation of cells labeled by the anti-CD8-PEO-Biotin/anti-Biotin-MicroBead system according to the invention with biotin leads to a significantly better release of the magnetic label in comparison to the anti-CD8-LC-LC-Biotin/anti-Biotin-MicroBead system. This example shows the possibility to efficiently release the magnetic labeling of separated cells in the anti-CD8-PEO-Biotin/anti-Biotin-MicroBead system by the addition of biotin as release agent.

Example 7 - Magnetic cell separation with anti-CD8-PEO-Biotin conjugate:

PBMCs in PBS/EDTA/BSA-buffer were incubated for 10 min at 4 °C with anti-CD8-LC-LC-Biotin (3) and anti-CD8-PEO-Biotin (4). The cells were washed with cold PBS/EDTA/BSA-buffer and incubated for 15 min at 4 °C with anti-Biotin-MicroBeads (Miltenyi Biotec GmbH) and for 5 min at 4°C with anti-CD8-PE. The cells were washed with cold PBS/EDTA-BSA-buffer and resuspended in 500 μL of cold buffer. The cell suspension was applied on a MS-column in a magnetic field for magnetic cell separation. The flow-through was collected as magnetically unlabeled cell fraction. The cells were washed within the magnetic field and the column was removed from the separator prior to elution of the cells with 1 mL of cold PBS/EDTA/BSA-buffer. The isolated cell fraction was incubated for 10 min at different concentrations of streptavidin with and without compound 12. The cell suspension was applied onto a second column and flow-through was collected as eluted cells. Cells retained by the column were eluted with 500 μL of cold PBS/EDTA/BSA-buffer in absence of the magnetic field. The amount of CD8 positive cells in the isolated fractions was determined by flow cytometry analysis. The percentage allocation of the eluted cells was calculated as follows:

$$\frac{\text{amount of cells in the eluted fraction}}{\text{amount of cells in the eluted fraction} + \text{amount of cells in the fraction retained by magnetic field}}$$

Fig. 8 illustrates that the incubation of cells labeled by the anti-CD8-PEO-Biotin/anti-Biotin-MicroBead system according to the invention with streptavidin provides a significantly faster release of the magnetic label in comparison to the anti-CD8-LC-LC-Biotin/anti-Biotin-MicroBead system. Moreover, the addition of compound 12 to streptavidin as release agent further improves the dissociation of the CD8-PEO-Biotin/anti-Biotin-MicroBead system. This example shows that the use of an auxiliary release agent according to the invention can significantly improve the release efficiency.

Example 8 - Magnetic cell separation with anti-CD8-PEO-Biotin conjugate:

PBMCs in PBS/EDTA/BSA-buffer were incubated for 10 min at 4 °C with anti-CD8-LC-LC-Biotin (3) and anti-CD8-PEO-Biotin (4). The cells were washed with cold PBS/EDTA/BSA-buffer and incubated for 15 min at 4 °C with anti-Biotin-MicroBeads and for 5 min at 4 °C with anti-CD8-PE. The cells were washed with cold PBS/EDTA-BSA-buffer and resuspended in 500 μL of cold buffer. The cell suspension was applied on a MS-column in a magnetic field for magnetic cell separation. The flow-through was collected as magnetically unlabeled cell fraction. The cells were washed within

the magnetic field and the column was removed from the separator prior to elution of the cells with 1 mL of cold PBS/EDTA/BSA-buffer. The isolated cell fraction was incubated for 10 min at different concentrations of APC-streptavidin with and without compound 12. The cell suspension was applied onto a second column and flow-through was collected as eluted cells. Cells retained by the column were eluted with 500 μL of cold PBS/EDTA/BSA-buffer in absence of the magnetic field. The amount of CD8 positive cells in the isolated fractions was determined by flow cytometry analysis. The percentage allocation of the eluted cells was calculated as follows:

$$\frac{\text{amount of cells in the eluted fraction}}{\text{amount of cells in the eluted fraction} + \text{amount of cells in the fraction retained by magnetic field}}$$

Fig. 9 illustrates that cells labeled by the anti-CD8-PEO-Biotin/anti-Biotin-MicroBead system according to the invention and treated with APC-streptavidin as release agent are significantly faster eluted from the magnetic field in comparison to cells labeled with the anti-CD8-LC-LC-Biotin/anti-Biotin-MicroBead system. Moreover, the addition of compound 12 to APC-streptavidin as release agent further improves the dissociation of the CD8-PEO-Biotin/anti-Biotin-MicroBead system. These results demonstrate that the use of auxiliary release agent according to the invention can significantly improve the release efficiency.

## Claims

1. Method for the separation of target cells from a cell sample comprising the steps:

   a) incubation of the cell sample with a releasable conjugate comprising a biotinylated ligand having a biotin moiety, a ligand moiety ($Ligand_1$) and a biotin-binding molecule (bbm) bound to the biotin moiety of the biotinylated ligand, wherein the biotin moiety and the ligand moiety of the biotinylated ligand are separated by a spacer group consisting of polyethylene glycol according to the general formula I

   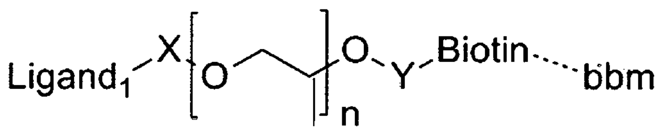

   I

   with n = 1 - 500, and X, Y = same or different, substituted alkyl groups having 1 to 20 carbon atoms with amine, amide, and/or thioether residues,
   wherein the ligand is a first antibody for which the target cells express an antigen, and the biotin-binding molecule (bbm) is a second antibody which comprises a magnetic particle as solid support,
   b) separation of the cells labeled with the a solid support obtained in step a) from the cell sample by applying an magnetic field,
   c) adding a release agent in a sufficient concentration to displace the biotin-binding molecule (bbm) from the biotin moiety of the biotinylated ligand.

2. Method according to claim 1 **characterized in that** subsequent to step c), in an optionally step d) the biotin-binding molecule (bbm) is separated from the target cells.

3. Method according to claim 2 **characterized in that** step d) is conducted by applying a magnetic field.

4. Method according to any of the claims 1 to 3 **characterized in that** step b) and c) are conducted in at least one column containing ferromagnetic material.

5. Method according to any of the claims 1 to 4 **characterized in that** the ligand comprises a chromophor unit, a fluorescence unit, or a radioactive unit as detection means.

6. Method according to any of the claims 1 to 5 **characterized in that** the detection means of the ligand and/or the biotin-binding molecule (bbm) is detected subsequent to step c) to detect the target cells.

7. Method according to any of the claims 1 to 6 **characterized in that** the ligand is an antibody or a fragment of an

antibody directed against antigen expressed intracellular or extracellular.

**8.** Method according to any of the claims 1 to 7, **characterized in that** the releasable conjugate has the general formula IIa, IIb, or IIc

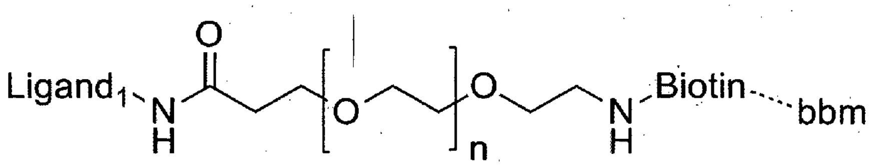

IIa

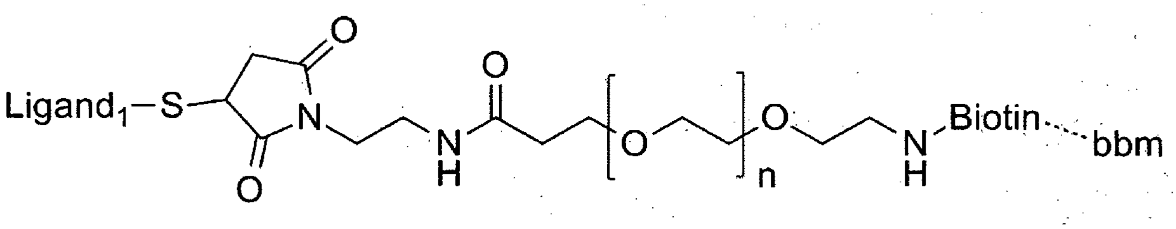

IIb

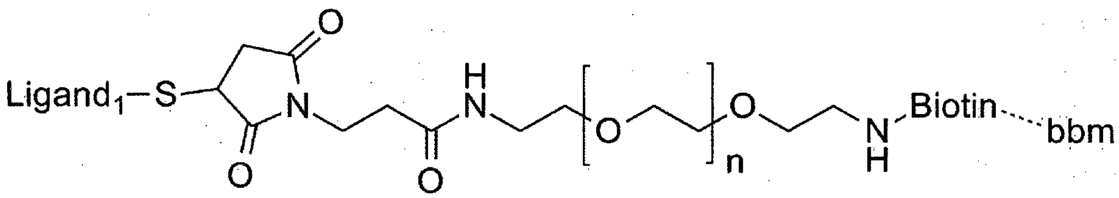

IIc

**9.** Method according to any of the claims 1 to 8, **characterized in that** the release agent is biotin or streptavidin.

**10.** Method according to any of the claims 1 to 9 **characterized in that** in step c), an auxiliary release agent having the general formulas VII, VIII, or IX

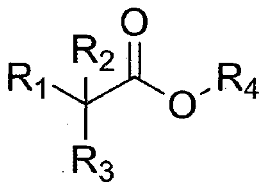

VII

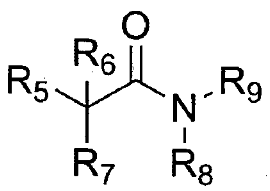

VIII

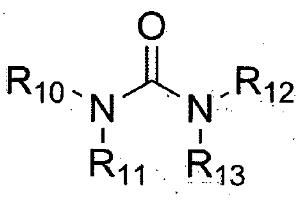

IX

with $R_{1-3,5-13}$ = same or different hydrogen, or substituted or unsubstituted alkyl residues having 1 - 20 carbon atoms and $R_4$ = substituted or unsubstituted alkyl residues, having 1 - 20 carbon atoms is additionally provided.

**Patentansprüche**

1. Verfahren zur Abtrennung von Zielzellen aus einer Zellprobe, das folgende Schritte umfasst:

   a) Inkubieren der Zellprobe mit einem freisetzbaren Konjugat, das einen biotinylierten Liganden umfasst, der einen Biotinanteil, einen Ligandenanteil ($Ligand_1$) und ein Biotin-bindendes Molekül aufweist, das an den Biotinanteil des biotinylierten Liganden gebunden ist, wobei der Biotinanteil und der Ligandenanteil des biotinylierten Liganden durch eine Spacergruppe getrennt sind, die aus Polyethylenglykol gemäß der allgemeinen Formel I besteht

   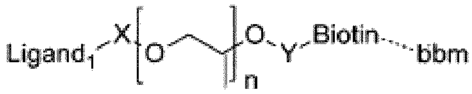

   I

   wobei n = 1-500 und X, Y = gleich oder verschieden voneinander, substituierte Alkylgruppen, die 1 bis 20 Kohlenstoffatome mit Amin- Amid- und/oder Thioetherresten aufweisen,
   wobei der Ligand ein erster Antikörper ist, für den die Zielzellen ein Antigen exprimieren, und das Biotin-bindende Molekül (bbm) ein zweiter Antikörper ist, der ein magnetisches Teilchen als festen Träger umfasst,
   b) Abtrennung der mit dem festen Träger markierten Zellen, die in Schritt a) erhalten wurden aus der Zellprobe durch Einsatz eines magnetisches Feldes,
   c) Hinzufügen einer Freisetzungssubstanz in einer ausreichenden Konzentration, um das Biotin-bindende Molekül (bbm) aus dem Biotinanteil des biotinylierten Liganden zu verdrängen.

2. Verfahren nach Anspruch 1, **dadurch gekennzeichnet, dass** im Anschluss an Schritt c) in einem optionalen Schritt d) das Biotin-bindende Molekül (bbm) von den Zielzellen abgetrennt wird.

3. Verfahren nach Anspruch 2, **dadurch gekennzeichnet, dass** Schritt d) durch Einsatz eines magnetischen Feldes durchgeführt wird.

4. Verfahren nach einem der Ansprüche 1 bis 3, **dadurch gekennzeichnet, dass** Schritt b) und c) in mindestens einer Säule durchgeführt werden, die ferromagnetisches Material enthält.

5. Verfahren nach einem der Ansprüche 1 bis 4, **dadurch gekennzeichnet, dass** der Ligand eine Chromphoreinheit, eine Fluoreszenzeinheit oder eine radioaktive Einheit als Nachweismittel umfasst.

6. Verfahren nach einem der Ansprüche 1 bis 5, **dadurch gekennzeichnet, dass** das Nachweismittel des Liganden und/oder des Biotin-bindenden Moleküls (bbm) im Anschluss an Schritt c) nachgewiesen wird, um die Zielzellen nachzuweisen.

7. Verfahren nach einem der Ansprüche 1 bis 6, **dadurch gekennzeichnet, dass** der Ligand ein Antikörper oder ein Fragment eines Antikörpers ist, der gegen ein intrazellulär oder extrazellulär exprimiertes Antigen gerichtet ist.

8. Verfahren nach einem der Ansprüche 1 bis 7, **dadurch gekennzeichnet, dass** das freisetzbare Konjugat die allgemeine Formel IIa, IIb oder IIc aufweist

   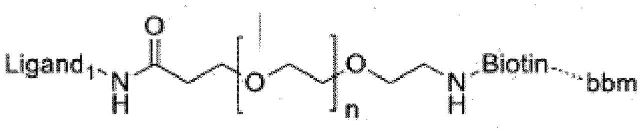

   IIa

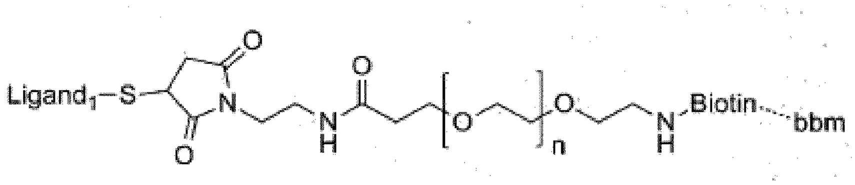

IIb

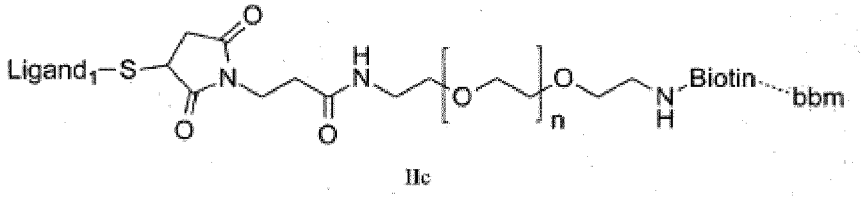

IIc

9. Verfahren nach einem der Ansprüche 1 bis 8, **dadurch gekennzeichnet, dass** die Freisetzungssubstanz Biotin oder Streptavidin ist.

10. Verfahren nach einem der Ansprüche 1 bis 9, **dadurch gekennzeichnet, dass** in Schritt c) zusätzlich eine unterstützende Freisetzungssubstanz vorgesehen wird, welche die allgemeine Formel VII, VIII oder IX aufweist

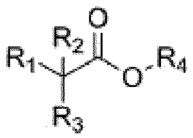

VII

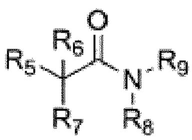

VIII

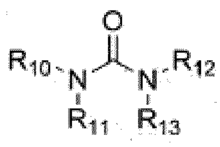

IX

mit $R_{1\text{-}3,5\text{-}13}$ - gleich oder verschieden voneinander Wasserstoff oder substituierte oder unsubstituierte Alkylreste, die 1-20 Kohlenstoffatome aufweisen und $R_4$ = substituierte oder unsubstituierte Alkylreste, die 1-20 Kohlenstoffatome aufweisen.

## Revendications

1. Procédé de séparation de cellules cibles à partir d'un échantillon cellulaire comprenant les étapes suivantes :

(a) l'incubation de l'échantillon cellulaire à l'aide d'un conjugué libérable comprenant un ligand biotinylé ayant un fragment biotine, un fragment ligand ($Ligand_1$) ainsi qu'une molécule de liaison à la biotine (bbm) liée au fragment biotine du ligand biotinylé, où le fragment biotine et le fragment ligand du ligand biotinylé sont séparés par un groupe d'espacement constitué de polyéthylène glycol, selon la formule I générale :

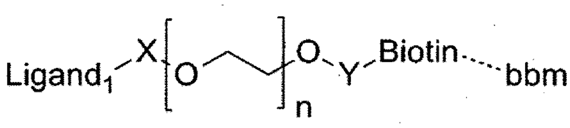

I

où n = 1-500 et X, Y = groupes alkyles substitués, identiques ou différents, possédant 1 à 20 atomes carbone avec des résidus d'amine, d'amide et/ou de thioéther, où le ligand est un premier anticorps pour lequel les cellules cibles expriment un antigène et la molécule de liaison à la biotine (bbm) est un second anticorps comprenant une particule magnétique comme support solide,
(b) la séparation des cellules étiquetées avec le support solide obtenues lors de l'étape a) à partir de l'échantillon cellulaire grâce à l'application d'un champ magnétique,

(c) l'ajout d'un agent de libération dans une concentration suffisante pour permettre le déplacement de la molécule de liaison à la biotine (bbm) à partir du fragment biotine du ligand biotinylé.

2. Procédé selon la revendication 1 **caractérisé en ce que**, suite à l'étape c), au cours d'une étape d) facultative, la molécule de liaison à la biotine (bbm) est séparée des cellules cibles.

3. Procédé selon la revendication 2 **caractérisé en ce que** l'étape d) est réalisée grâce à l'application d'un champ magnétique.

4. Procédé selon l'une quelconque des revendications 1 à 3 **caractérisé en ce que** les étapes b) et c) sont réalisées dans au moins une colonne contenant un matériau ferromagnétique.

5. Procédé selon l'une quelconque des revendications 1 à 4 **caractérisé en ce que** le ligand comprend une unité chromophore, une unité fluorescente ou une unité radioactive comme moyen de détection.

6. Procédé selon l'une quelconque des revendications 1 à 5 **caractérisé en ce que** le moyen de détection du ligand et/ou de la molécule de liaison à la biotine (bbm) est détecté suite à l'étape c) afin de détecter les cellules cibles.

7. Procédé selon l'une quelconque des revendications 1 à 6 **caractérisé en ce que** le ligand est un anticorps ou un fragment d'anticorps dirigé contre l'antigène exprimé à l'intérieur ou à l'extérieur de la cellule.

8. Procédé selon l'une quelconque des revendications 1 à 7 **caractérisé en ce que** le conjugué libérable possède la formule générale IIa, IIb ou IIc :

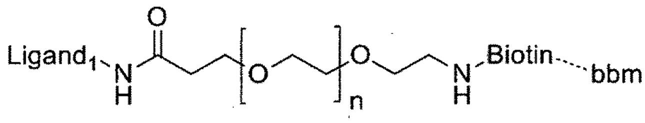

IIa

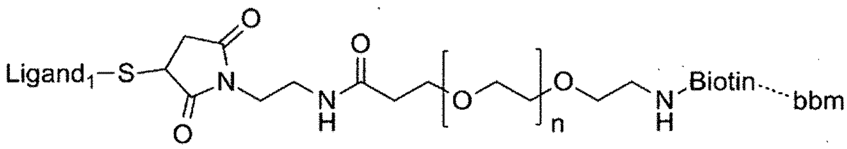

IIb

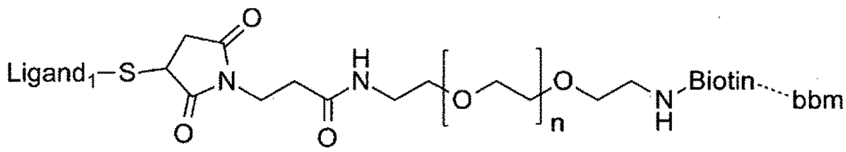

IIc

9. Procédé selon l'une quelconque des revendications 1 à 8 **caractérisé en ce que** l'agent de libération est la biotine ou la streptavidine.

10. Procédé selon l'une quelconque des revendications 1 à 9 **caractérisé en ce que** dans l'étape c), un agent de libération auxiliaire ayant la formule générale VII, VIII ou IX

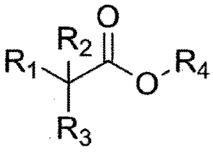 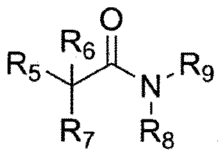 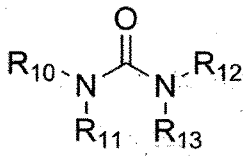

VII VIII IX

où $R_{1-3,5-13}$ = résidus d'hydrogène ou alkyles substitués ou non substitués, identiques ou différents, possédant 1 à 20 atomes carbone et $R_4$ = résidus alkyles substitués ou non substitués possédant 1 à 20 atomes carbone, est fourni en plus.

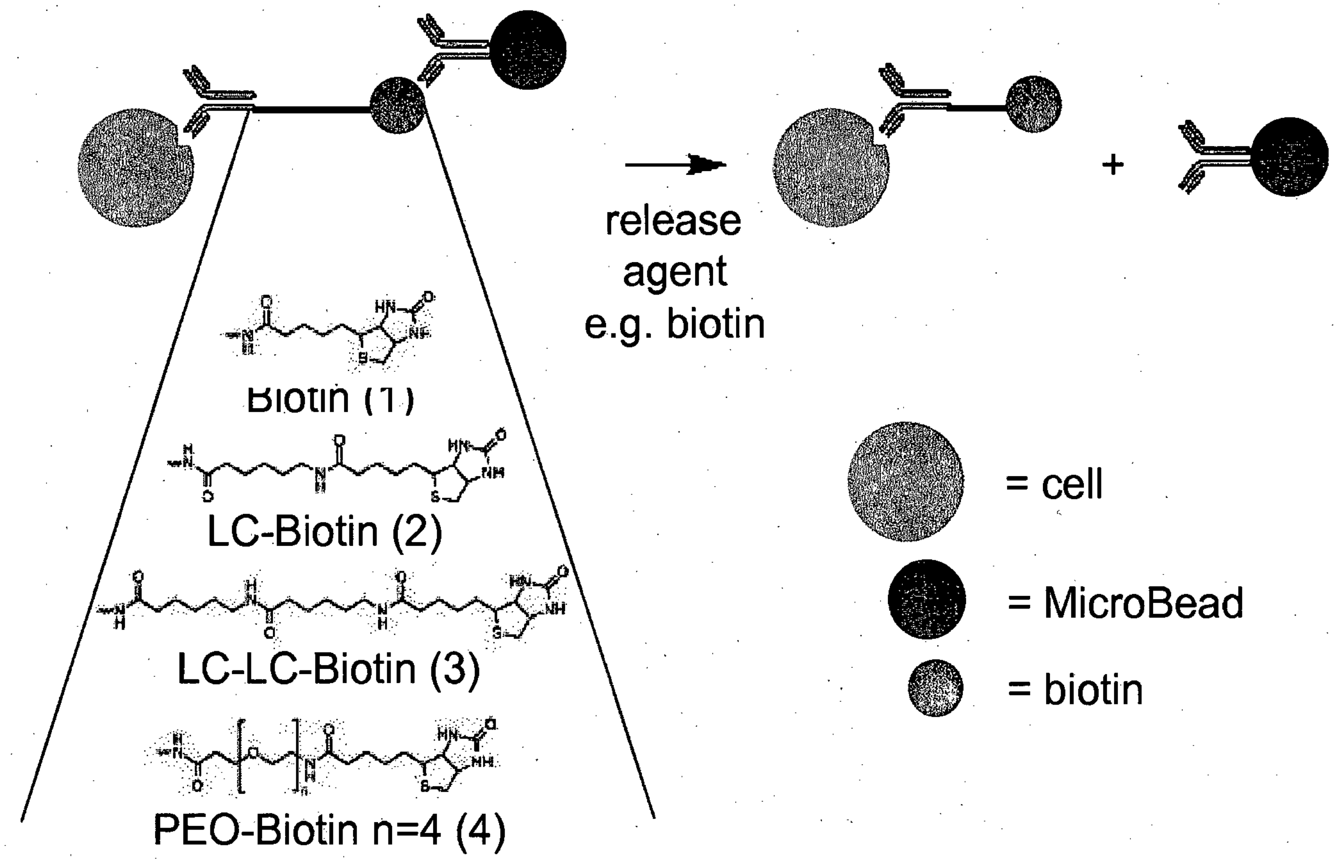
release
agent
e.g. biotin
+
Biotin (1)
LC-Biotin (2)
LC-LC-Biotin (3)
PEO-Biotin n=4 (4)
= cell
= MicroBead
= biotin

Fig.1

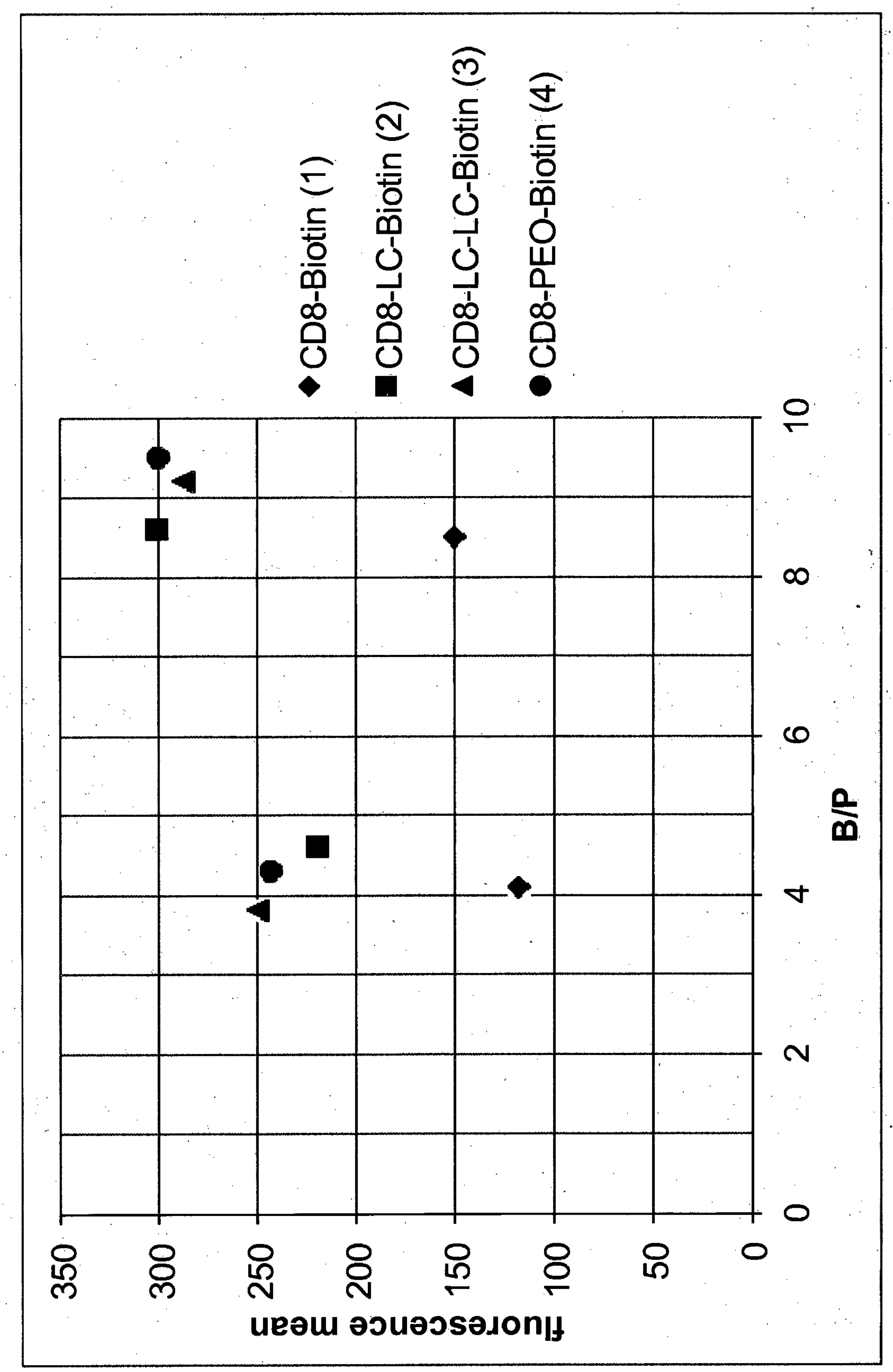

CD8-Biotin (1)
CD8-LC-Biotin (2)
CD8-LC-LC-Biotin (3)
CD8-PEO-Biotin (4)
fluorescence mean
350
300
250
200
150
100
50
0
B/P
0
2
4
6
8
10


Fig. 2:

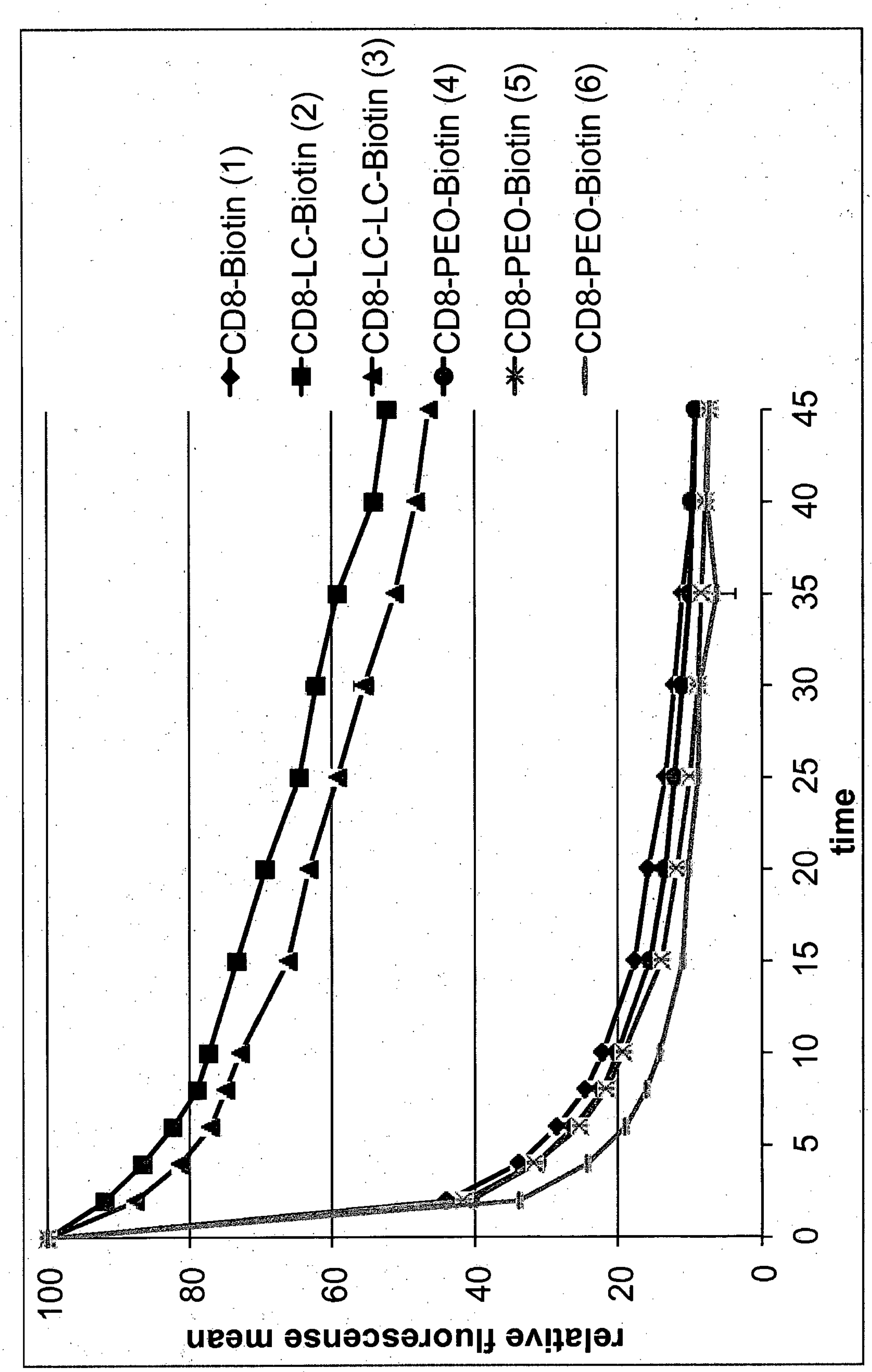
CD8-Biotin (1)
CD8-LC-Biotin (2)
CD8-LC-LC-Biotin (3)
CD8-PEO-Biotin (4)
CD8-PEO-Biotin (5)
CD8-PEO-Biotin (6)
relative fluorescense mean
100
80
60
40
20
0
0
5
10
15
20
25
30
35
40
45
time

Fig. 3:

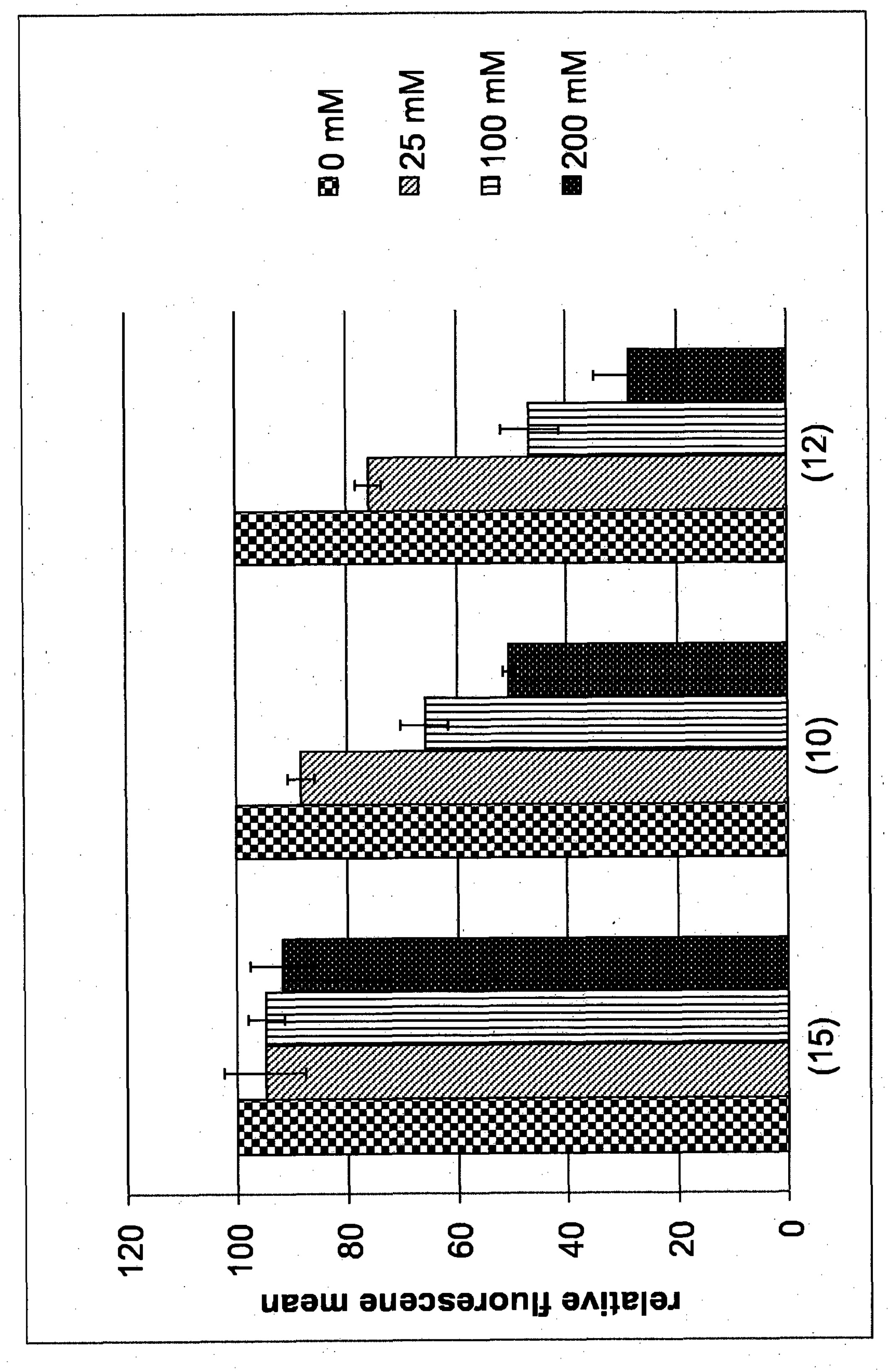
relative fluorescene mean
120
100
80
60
40
20
0
(15)
(10)
(12)
0 mM
25 mM
100 mM
200 mM

Fig. 4:

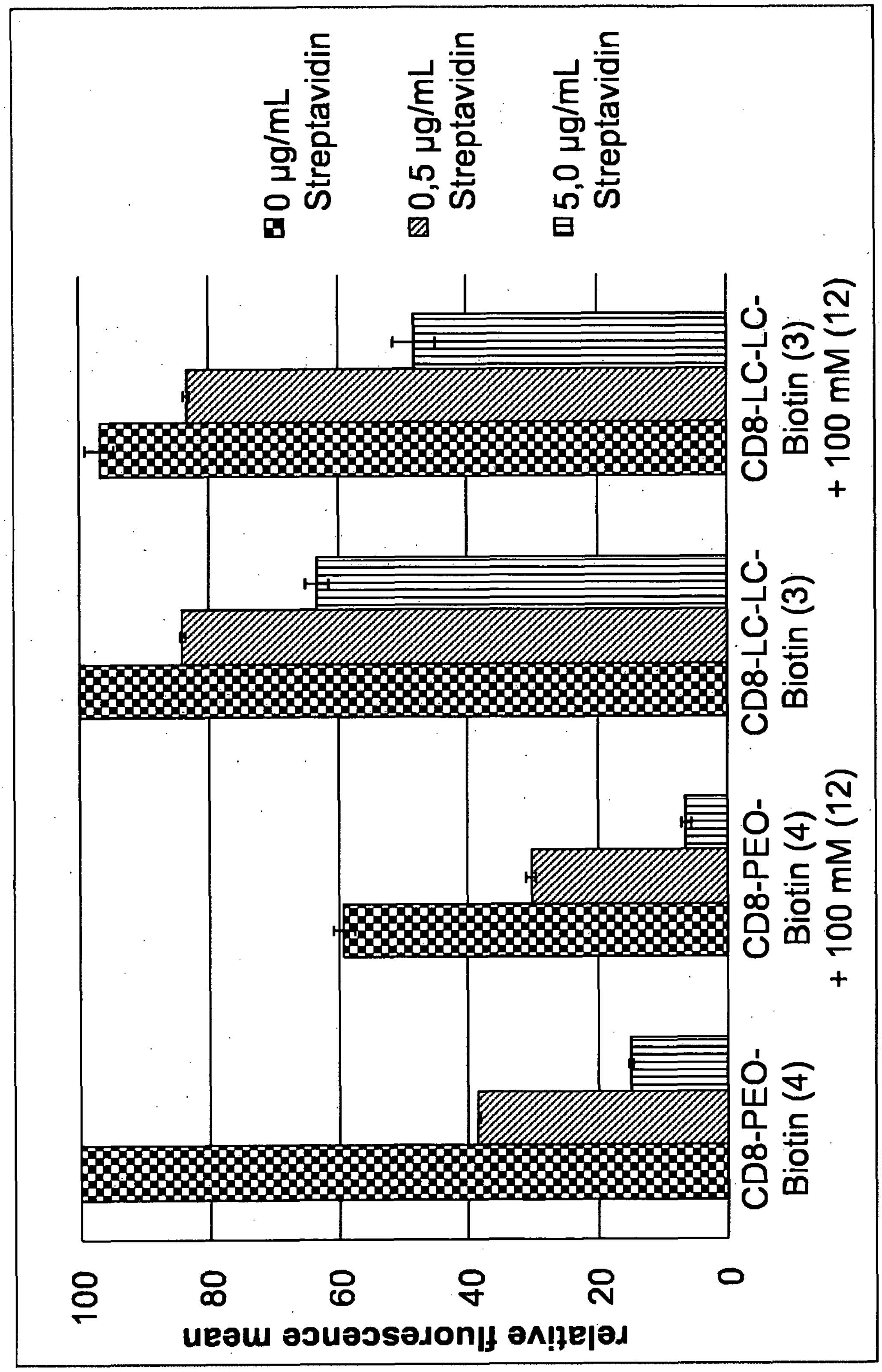
relative fluorescence mean
100
80
60
40
20
0
CD8-PEO-Biotin (4)
CD8-PEO-Biotin (4) + 100 mM (12)
CD8-LC-LC-Biotin (3)
CD8-LC-LC-Biotin (3) + 100 mM (12)
0 µg/mL Streptavidin
0,5 µg/mL Streptavidin
5,0 µg/mL Streptavidin

Fig. 5:

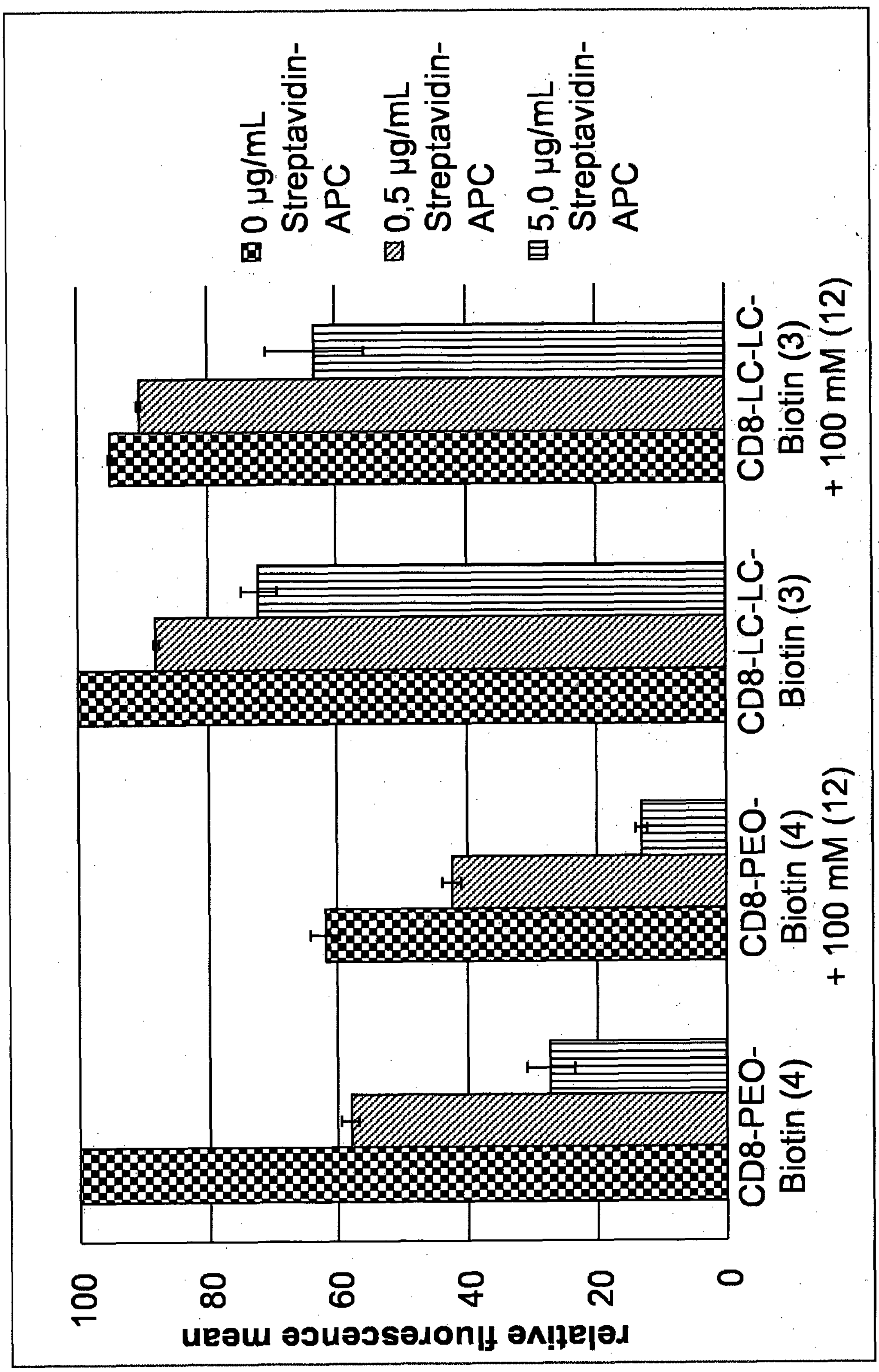
relative fluorescence mean
100
80
60
40
20
0
CD8-PEO-Biotin (4)
CD8-PEO-Biotin (4) + 100 mM (12)
CD8-LC-LC-Biotin (3)
CD8-LC-LC-Biotin (3) + 100 mM (12)
0 µg/mL Streptavidin-APC
0,5 µg/mL Streptavidin-APC
5,0 µg/mL Streptavidin-APC

Fig. 6:

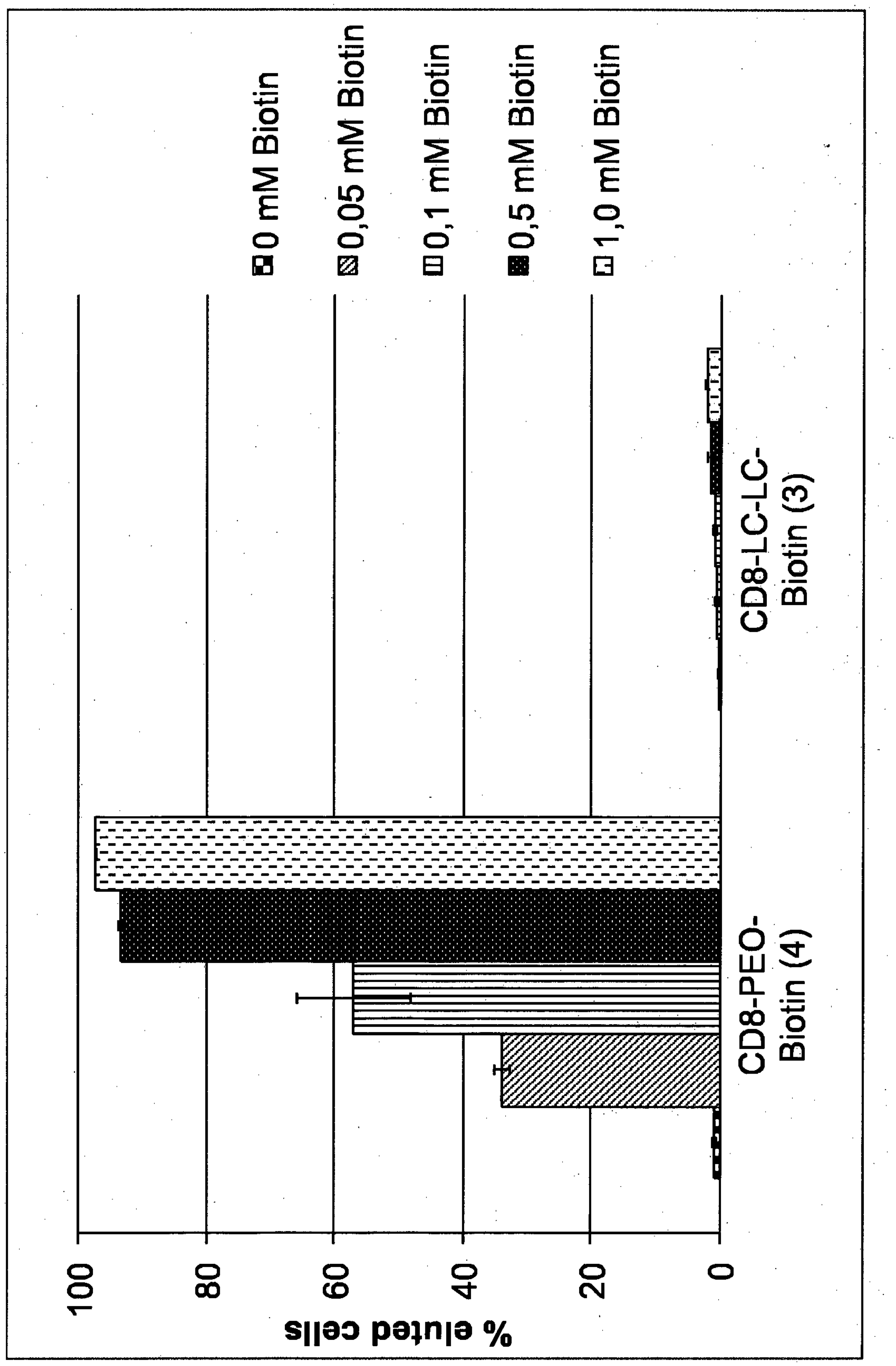
0 mM Biotin
0,05 mM Biotin
0,1 mM Biotin
0,5 mM Biotin
1,0 mM Biotin
% eluted cells
100
80
60
40
20
0
CD8-PEO-Biotin (4)
CD8-LC-LC-Biotin (3)

Fig. 7:

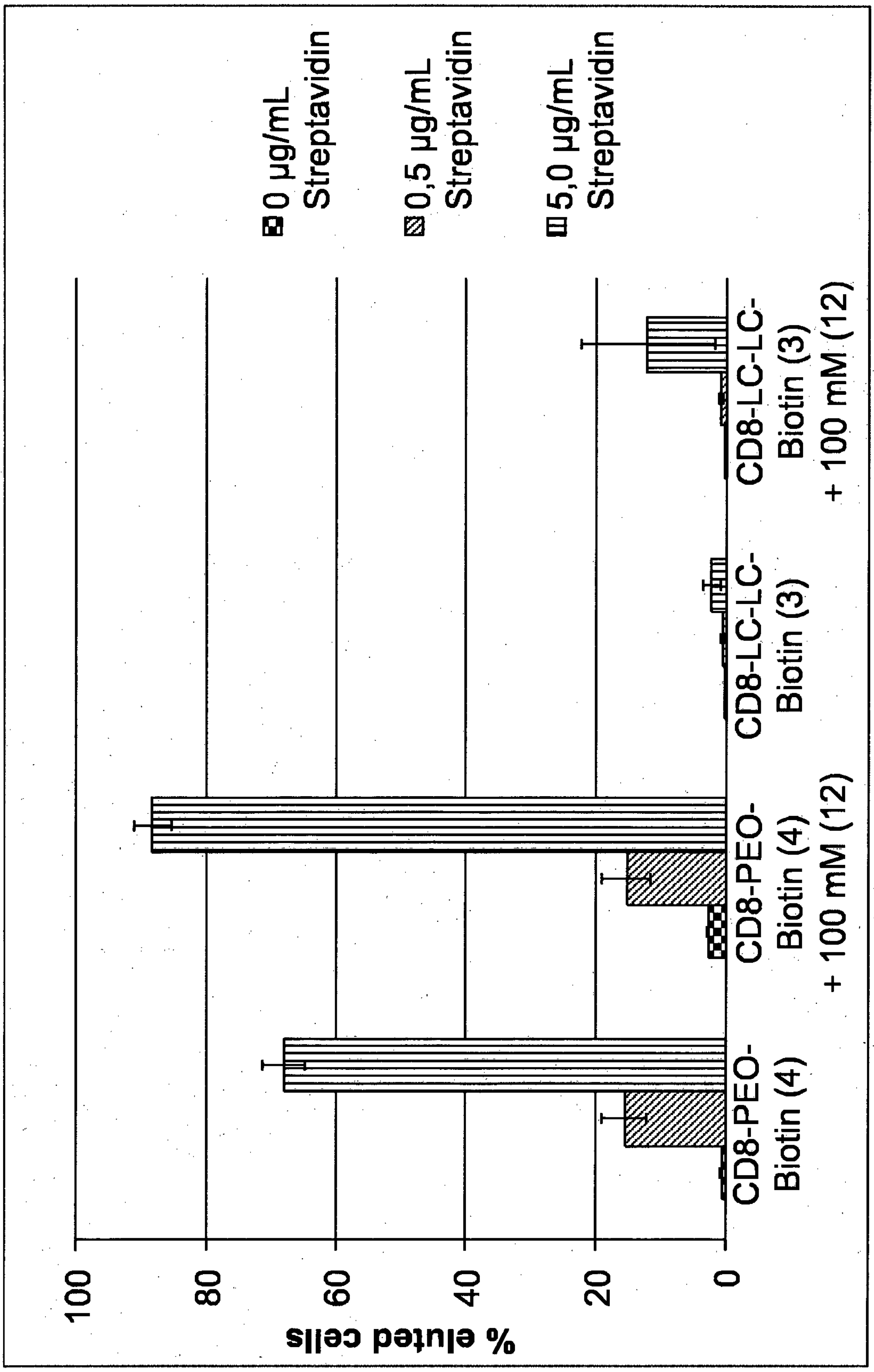
% eluted cells
100
80
60
40
20
0
CD8-PEO-Biotin (4)
CD8-PEO-Biotin (4) + 100 mM (12)
CD8-LC-LC-Biotin (3)
CD8-LC-LC-Biotin (3) + 100 mM (12)
0 µg/mL Streptavidin
0,5 µg/mL Streptavidin
5,0 µg/mL Streptavidin

Fig. 8:

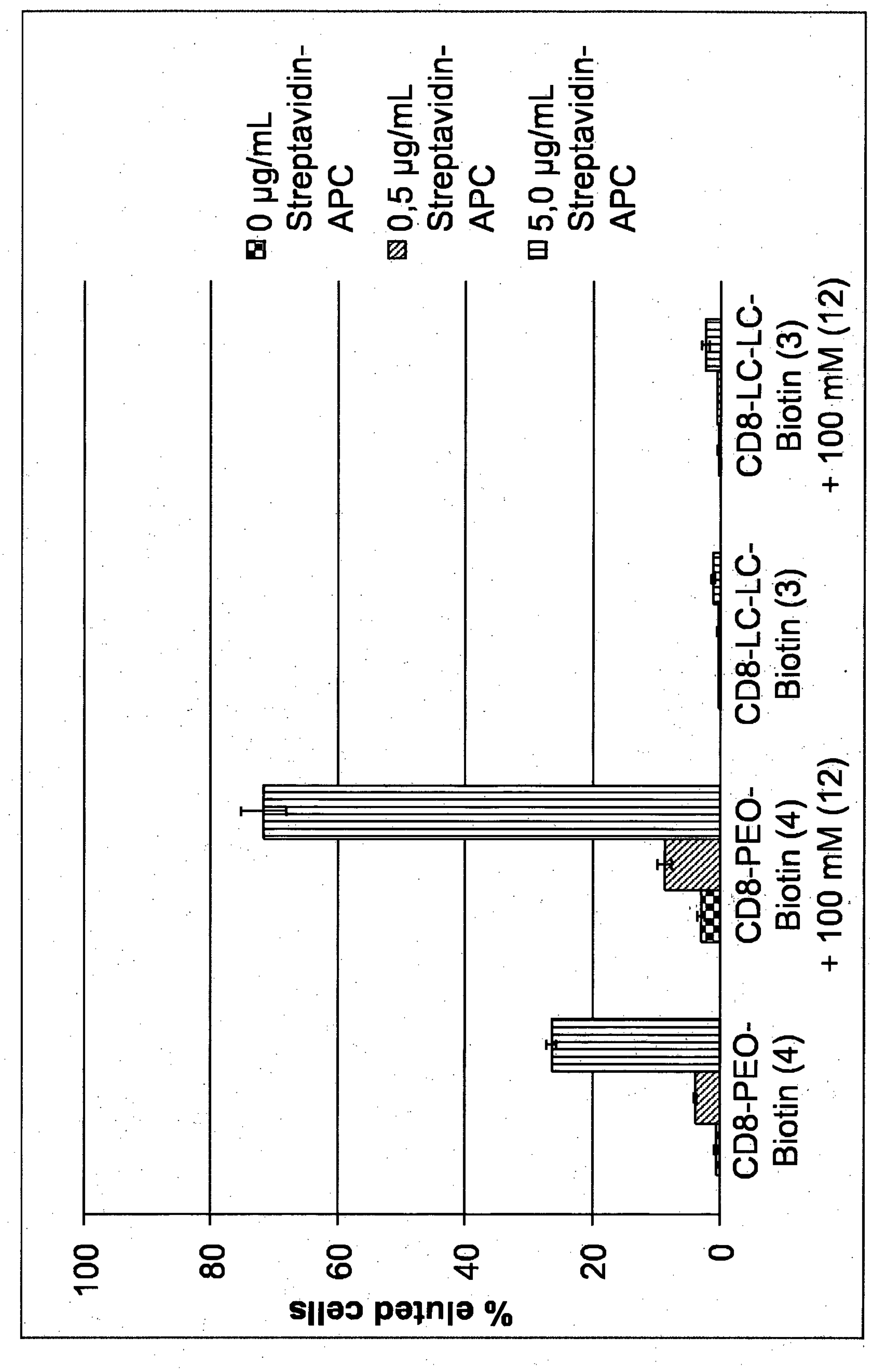
% eluted cells
100
80
60
40
20
0
CD8-PEO-Biotin (4)
CD8-PEO-Biotin (4) + 100 mM (12)
CD8-LC-LC-Biotin (3)
CD8-LC-LC-Biotin (3) + 100 mM (12)
0 µg/mL Streptavidin-APC
0,5 µg/mL Streptavidin-APC
5,0 µg/mL Streptavidin-APC

Fig. 9:

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20080255004 A
- US 5506121 A
- US 5215927 A
- WO 9216841 A
- US 6869606 B
- US 4656252 A
- US 5518882 A


### Non-patent literature cited in the description


- **M. QURESHI et al.** *J. Biol. Chem.,* 2001, vol. 276, 46422-46428
- **T. SANO et al.** *Proc.Natl. Acad. Sci. USA,* 1995, vol. 92, 3180-3184
- **JAMES HIRSCH et al.** *Analytical Biochemistry,* 2002, vol. 308, 343-357
- **KE SHAN et al.** Avidin-Biotin-PEG-CPA Complexes as Potential EPR-Directed Therapeutic Protein Carriers: Preparation and Characterization. *Bioconjugate Chemistry,* 01 September 2007, vol. 18 (5), 1644-1650
- Intermolecular interaction of avidin and PEGylated biotin. *Bioconjugate Chemistry,* 21 November 2007, vol. 18 (6), 2109-2114
- **RAMPERSAUD et al.** Novel discrete PEG-based crosslinking reagents for conjugation of antibodies and proteins to biotin, fluorochromes, enzymes and gold that eliminate aggregation, improves solubility, reduces non-specific binding and enhances low level detection limits. *MOLECULAR BIOLOGY OF THE CELL,* 2011, vol. 22, 1931